# EUROPEAN PATENT APPLICATION

(11) **EP 1 779 838 A1**
(43) Date of publication of application: **02.05.2007**
(21) Application number: 05023335.2
(22) Date of filing: 26.10.2005
(51) Int. Cl.: A61K 8/34, A61K 8/81, A61K 8/41, A61K 8/39, A61Q 5/12, A61Q 5/02

(54) **Hair conditioning composition comprising Polyquaternium 37, fatty alcohol and non-ionic / cationic surfactant**

(71) Applicant: KPSS-Kao Professional Salon Services GmbH, 64297 Darmstadt (DE)
(72) Inventor: Hoffmann, Martin, 64673 Zwingenberg (DE); Ast, Andrea, 64283 Darmstadt (DE)

(57) **Abstract**

The present invention relates to a hair conditioning composition with excellent body enhancing and volume up effect especially for fine hair and also gives hair excellent combability, elasticity and shine. The inventors of the present application has surprisingly found out that a composition comprising polyquaternium-37 as a cationic polymer and an instable emulsion composition comprising at least one fatty alcohol and at least one surfactant selected from cationic and non-ionic ones at a weight ratio of cationic and/or non-ionic surfactants to fatty alcohol in the range of 1: 2 to 1:10, wherein cationic surfactants are selected from compounds according to general formula 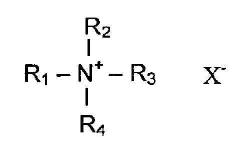 where R₁ is a saturated or unsaturated, branched or non-branched alkyl chain with 8-22 C atoms or

R₅ CO NH (CH₂)ₙ

where R₅ is saturated or unsaturated, branched or non-branched alkyl chain with 7-21 C atoms and n has value of 1 - 4,
or

R₆ CO O (CH₂)ₙ

where R₆ is saturated or unsaturated, branched or non-branched alkyl chain with 7-21 C atoms and n has value of 1 - 4, and
R₂, R₃ and R₄ are H or lower alkyl or hydroxyalkyl or polyhydroxyalkyl with 1 to 4 C atoms and X is chloride, bromide, methosulfate,
enhances excellently hair body and volume as well as combability, elasticity and shine. The important benefit of the compositions of the present invention that no weigh down effect is observed, which obviously results in loss on volume and body.

## Description

The present invention relates to a hair conditioning composition with excellent body giving and volume up effects, especially for fine hair and also gives hair excellent combability, elasticity and shine.

Hair conditioners of various types either in application or in the form of preparation types have found their wide range of usage in hair dressing practice. Among those rinse off types of conditioners have always been preferred in hair care. Usually, rinse-off type conditioners are in the form of emulsions and comprising fatty alcohols and emulsifiers of different character as the main principal ingredients. In addition, they certainly comprise conditioning ingredients of various types and those of common ingredients in cosmetic preparations. A general overview on the known hair conditioning products and also their usual compositions can be found in the monography of K. Schrader, "Grundlage und Rezepturen der Kosmetika", 2nd Ed. 1989, pp 722 - 781.

The people with fine hair always refer to hair volume and body as the main hair problem when they use conventional conditioning products especially with rinse off usage. Therefore, these people usually refrain from using such rinse off conditioning products and this behaviour causes further problems such as loss of combability, elasticity and shine. With the usage of products designed for leave in application, some of the problems might temporarily be helped, but an acceptable solution to the daily hair care benefits remains up until now not offered.

The present inventions starts form the above problems and aims at effectively solving these problems of volume and body, especially for fine hair, with the compositions further described in detail below in the description

The inventors of the present application has surprisingly found out that a composition comprising polyquaternium-37 as a cationic polymer and an instable emulsion composition comprising at least one fatty alcohol and at least one surfactant selected from cationic and non-ionic ones at a weight ratio of cationic and/or non-ionic surfactants to fatty alcohol in the range of 1: 2 to 1:10 enhances excellently hair body and volume as well as combability, elasticity and shine of hair, especially fine hair. The important benefit of the compositions of the present invention that no weigh down effect is observed, which obviously results in loss on volume and body.

Accordingly, the first object of the present invention is a conditioning composition comprising Polyquaternium 37 at a concentration of 0.1 to 5% by weight, calculated to total composition, and an instable emulsion composition comprising at least one fatty alcohol at a concentration of 0.1 to 2.5% by weight, calculated to total composition, and at least one surfactant selected from non-ionic and cationic ones at a weight ratio of cationic and/or non-ionic surfactants to fatty alcohol in the range of 1: 2 to 1:10, wherein cationic surfactant (quaternary ammonium compound) is selected from compounds according to general formula 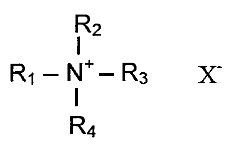 where R₁ is a saturated or unsaturated, branched or non-branched alkyl chain with 8-22 C atoms or

R₅ CO NH (CH₂)ₙ

where R₅ is saturated or unsaturated, branched or non-branched alkyl chain with 7-21 C atoms and n has value of 1 - 4,
or

R₆ CO O (CH₂)n

where R₆ is saturated or unsaturated, branched or non-branched alkyl chain with 7-21 C atoms and n has value of 1 - 4, and
R₂ , R₃ and R₄ are H or lower alkyl or hydroxyalkyl or polyhydroxyalkyl with 1 to 4 C atoms and X is chloride, bromide, methosulfate.

Further, object of the present invention is the use of the composition as defined above for giving hair body and volume, especially fine hair.

With the term instable emulsion it is meant that the emulsion is formed immediately after preparation, but it is not stable on storage, namely it separates after maximum one month storage at 40° into two phases.

The conditioning compositions of the present invention comprise cationic polymer polyquaternium-37 at a concentration of 0.1 to 5%, preferably 0.2 to 4%, more preferably 0.3 to 4% and most preferably 0.5 to 3% by weight calculated to total composition.

The instable emulsion comprises at least one fatty alcohol. The fatty alcohols preferred are linear or branched, saturated or unsaturated with 10 to 24, preferably 12 to 22 carbon atoms in its alkyl chain. Nonlimiting examples are myristyl alcohol, cetyl alcohol, stearyl alcohol, palmityl alcohol, oleyl alcohol, lauryl alcohol, coconut alcohol, palm alcohol, behenyl alcohol, arachidyl alcohol and their mixtures. Preferred are myristyl alcohol, cetyl alcohol, stearyl alcohol, palmityl alcohol, oleyl alcohol, behenyl alcohol and their mixtures.

Concentration of fatty alcohol in the compositions of present invention is between 0.1 and 2.5%, preferably between 0.2 and 2%, more preferably 0.3 to 2% and most preferably 0.5 to 1.5% by weight calculated to total composition. The concentrations mentioned refer to the total concentration of the fatty alcohols in the case that the composition comprises more than one fatty alcohol.

Hair conditioners of present invention comprise at least one surfactant selected form non-ionic and cationic ones. Nonionic surfactants are first of all C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16", "Ceteareth-20".

Further non-ionic surfactants may be used in the conditioners of the present invention are compounds from the category of alkyl polyglucosides with the general formula

R₇-0-(CH₂CH₂0)ₙ-Zₓ,

wherein R₇ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Hydrogenated castor oil with variable ethylene glycol units is also found to be suitable non-ionic surfactant. Those are for example known from BASF under the trade name Cremophor.

Further additionally useful surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain. Suitable amineoxides are on the market, for example, under the trade names "Ammonyx^{®}", "Aromox^{®}" or "Genaminox^{®}".

Suitable cationic surfactants have already been mentioned above with their general formula. Examples to cationic surfactants are particularly cetyl trimethly ammonium chloride, steartrimonium chloride, behentrimoinium chloride, stearamidopropyl trimonuim chloride, behenamidopropylethyldimonium ethosulfate, behenamidopropyltrimonium methosulfate, cocamidopropytrimonium chloride, cocotrimonim chloride, palmitamidopropyltrimonum chloride.

The concentration of surfactants in total, cationic and non-ionic surfactants, is should be in the range where the weight ratio of cationic and /or non-ionic surfactants to fatty alcohol is in the range of 1:2 to 1:10, preferably 1:2 to 1:8, more preferably 1:2 to 1:5. Conditioners of the present invention can also comprise non-ionic and cationic surfactants in combination.

Further optional surfactant components at minor concentration may be included into the conditioners of present invention are fatty acid mono- and dialkanolamides, such as coco fatty acid monoethanolamide and myristic fatty acid monoisopropanolamide.

Conditioners of the present invention may contain amphoteric or zwitterionic surfactants. Suitable ones are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 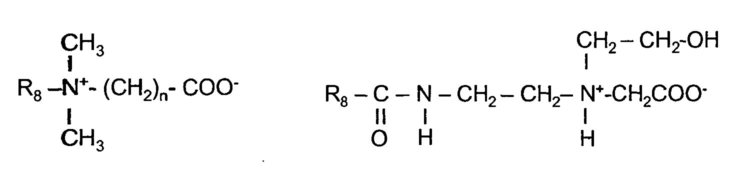 wherein R₈ is a C₈-C₁₈-alkyl group and n is 1 to 3,
sulfobetaines of the structure 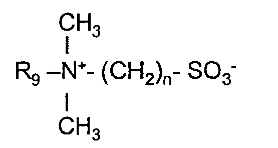 wherein R₉ is a C₈-C₁₈-alkyl group and n is 1 to 3,
and amidoalkyl betaines of the structure 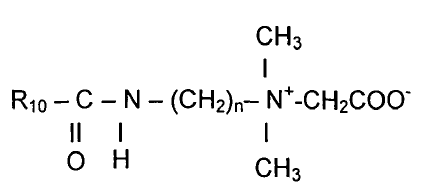 wherein R₁₀ is a C₈-C₁₈-alkyl group and n is 1 to 3,
Conditioner compositions of the present invention can contain additional hair conditioning agents selected from quaternary ammonium compounds, cationic polymers, silicone compounds, natural or synthetic oils, non-ionic conditioning agents and hair restructuring compounds.

On of the suitable conditioning agents are of those quaternary ammonium compounds according to the general structure given above (page 2) wherein R1 has the same meaning as above, and
R₂ is additionally unsaturated or saturated, branched or non-branched alkyl chain with 8 to 22 C atoms or

R₅ CO NH (CH₂)ₙ

or

R₆ CO O (CH₂)ₙ

where R₅ , R₆ and n are same as above, and
R₃ and R₄ are same as above.

Examples are distearyldimonium chloride, dipalmitoylethylhydroxyethylmonium chloride, dioleoylethyl dimethyl ammonium methosulfate, dioleoylethyl hydroxyethylmonium methosulfate, dilinolamidopropyldimonium chloride, dioleylethyl hydroxyethylmonium chloride, dipalmitoylethyldimonium chloride.

From the above quaternary ammonium compounds disclosed with the general formula, especially to mention are those compounds known **per se** and are on the market, for example, under the trade names "Schercoquat^{®}", "Dehyquart^{®} F30" and "Tetranyl^{®}". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially to mention are those compounds are known **per se** and on the market, for example, under the trade name "INCROQUAT^{®} HO" or "OCS". These compounds are known with a general ingredient category under "amidoquat" in the cosmetic industry.

Further hair conditioning agents suitable for compositions of the present invention are those of cationic polymers best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28. It has been found out that especially those of cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

The cationic polymers also include the quaternized products of graft polymers from organopolysiloxanes and polyethyl oxazolines described in EP-A 524 612 and EP-A 640 643.

Conditioner compositions of the present invention can optionally contain non-ionic polymers such as hydroxyethylcellulose, hydroxypropylclellulose, xanthan gum, xyloglucan, polyvinylalcohol, polyvinylpyrrolidone or their derivatives.

Anionic polymers should not be used in the conditioner compositions of the present invention as incompatibilities arose with the main thickening cationic polymer and other cationic conditioners. Typical example of those which should not be used is acrylate type of polymers know with the trade name Carbopol from Goodrich.

Oily substances as conditioning agents are selected from such as silicone oils, either volatile or non-volatile, natural and synthetic oils. Among silicone oils those can be added to the compositions include dimethicone and its derivatives, amodimethicone and its derivatives (cationic silicones), dimethiconol and its derivatives, polydimethylsiloxane and its derivatives, DC fluid ranges from Dow Corning. Natural oils suitable are such as olive oil, almond oil, avocado oil, weizenkeim oil, ricinus oil and the synthetic oils, such as mineral oil. Concentration of these oily ingredients should be 0.01 to 5%, preferably 0.05.to 4%, more preferably 0.1 to 2.5% by weight calculated to total composition.

In one of the preferred form of the present invention the compositions comprise at least one ester of aliphatic linear or branched carboxylic acid with a primary or secondary linear or branched alcohol at a concentration of 0.01 to 5%, preferably 0.05.to 4%, more preferably 0.1 to 2.5% by weight calculated to total composition. Examples include isopropyl myristate, palmitate stearate and isostearate, oleyl oleate, isocetyl stearate, hexyl laurate, dibutyl adipate, dioctyl adipate, myristyl myristate and oleyl erucate. The most preferred ones are isopropyl myristate, palmitate, stearate and isostearate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₁ CO (O CH₂ CH₂)ₙ OH

R₁₁ CO (O CH₂ CH₂)ₙ O OC R₁₂

where R₁₁ and R₁₂ are independent from each other saturated, unsaturated or branched or non-branched alkyl chain with 7 to 21 C atoms and n is typically 2 - 100.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

One of the known hair restructuring agents is ceramide type of compound with the general formula 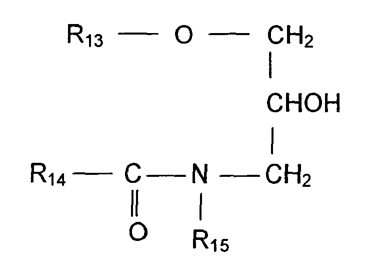 where R₁₃ and R₁₄ are independent from each other alkyl- or. alkenyl group with 10 to 22 carbon atoms, R₁₅ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide.

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1 % by weight calculated to the total weight of the composition. The fatty acids may be contained at a level of 0.01 to 1 % and epecially 0.01 to 0.5% by weight calculated to the total weight of the composition. Phytosterol concentration of the conditioners is less than 1% and preferably less than 0.5%, more preferably 0.05 to 3% by weight calculated to the total weight of the composition. It should be noted without limiting the use of those ingredients the effect of those hair restructuring ingredients is especially elevated when used in combination with penetration enhancers.

According to the invention, conditioning composition can comprises at least one direct acting dyestuff and be used as conditioning and colouring composition. The direct dyes referred are cationic, anionic, neutral and of plant dyes.

Suitable cationic dyestuffs are in principal those available on the market for hair colouring applications. Some examples to those are: Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51 Basic Red 76, Basic Violet 1, Basic Violet 2, Basic Violet 3, Basic Violet 10, Basic Violet 14 and Basic Yellow 57, Basic Orange 31 and Basic Yellow 87. For this purpose, special reference is made to the PCT application WO 95/15144 of Ciba-Geigy AG. All cationic dyes disclosed therein are included here by reference.

Cationic dyestuffs can be included into the compositions of the present invention at a concentration of 0.001 to 2%, preferably 0.005 to 1.5% and more preferably 0.01 to 1% by weight, calculated to total aqueous composition.

Anionic dyes may as well be used either alone or in combination with cationic direct dyes. The suitable ones are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

According to the invention, anionic dyes may be included at a concentration of 0.001 to 2%, preferably 0.005 to 1.5% and more preferably 0.01 to 1% by weight, calculated to total composition.

Additionally, the coloring compositions of the present invention may comprise neutral dyes (HC dyes), so called nitro dyes either alone or in addition to the cationic and/or anionic direct dyes. Concentration of those can typically be in the range of 0.001 to 2%, preferably 0.01 to 1.5% and more preferably 0.05 to 1 % by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No. 12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used in combination with cationic direct-acting dyestuffs, for example henna (red or black), alkanna root, laccaic acid, indigo, logwood powder, madder root and rhubarb powder, etc.

The conditioner compositions may contain organic solvents, for example, as penetration enhancer such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol. Concentration of organic solvents in the conditioner composition can be in the range from 0.1 to 10% by weight, preferably 0.1 to 7.5% by weight, and more preferably 0.1 to 5% by weight calculated to the total composition. The organic solvents may at the same time serve to solubilize ingredients which are not readily soluble in the conditioner composition.

Compositions of the present invention can comprise UV filters for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher.

The preferred amount of the UV-absorber ranges from about 0.01 % to 2.5%, more preferably from 0.05 % to 1 % by weight, calculated to the total composition.

Viscosity of conditioner compositions according to the present invention between 10,000 mPa.s to 60,000 mPa.s, preferably 15,000 mPa.s to 50,000 mPa.s, more preferably 20,000 to 40,000 mPa.s measured at 20°C with Brookfield viscosimeter with, for example, Spindle 5 at 5 rpm. The viscosity values are read after 30 seconds from the start of the measurement. In the selection of the viscosity, special attention must be paid to the way of application and packaging to be used. It should be noted that the viscosity of the conditioners is less sensitive to temperature fluctuations. In other words, the viscosity changes observed with either decreasing or increasing temperatures is relatively small when compared to well known gel type of preparations thickened such as with hydroxyethylcellulose.

The pH of the conditioners of the present invention varies from 2 to 7, particularly 2 to 6 and more particularly 2.5 to 5.0 and most preferably 3 to 4.5.

For adjusting the pH of the said conditioner compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be adjusted in a way that conditioner composition so obtained has a pH value between 2 to 7. Typically concentration for acids can be 0.01 - 5% by weight, preferably 0.01 - 4% by weight, more preferably 0.05 - 2.5% by weight calculated to the total composition. The pH of the conditioner composition can also be adjusted to the required pH by using alkaline solution such as sodium hydroxide, potassium hydroxide or their salts with those acids mentioned above in the case that at the selected acid concentration pH of the composition is lower than that of the aimed value.

The conditioner composition may contain moisturisers and natural ingredients showing hair conditioning benefits.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of almond, aloe, pineapple, artichoke, arnica, avocado, valerian, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cocoanut, mango, peach, lemon, cornflower, wheat, apricot, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon^{®}" products, "Herbasol^{R}", "Sedaplant^{R}" and "Hexaplant^{R}". Extracts and the preparation thereof are also described in "Hagers Handbuch der pharmazeutischen Praxis", 4^{th} Ed..

Among the natural ingredients in the form of an extract, especially preferred component of the composition according to the invention is green tea extract. This tea extract is obtained from the leaves, leaf buds and tender stems of the tea shrub, Camellia sinensis or Camellia oleifera, by aqueous or hydro-alcoholic extraction and subsequent spray-drying. In difference to black tea, green tea is a non-fermented product obtained from the Thea sinensis or Thea assamica species. An overview of the biological and pharmacological effects of green tea and the ingredients thereof can be found, e.g., in an article by A. Pistorius, "Seifen-Öle-Fette-Wachse-Journal", Volume 122., No. 7/1996, pages 468 to 471, to which reference is made. The content of green tea extract is variable in the compositions according to the invention. It preferably ranges from 0.01 % to 10 %, preferably 0.05 % to 5% by weight, calculated to the total composition and the pulverulent extract.

Natural ingredients extracts suitable are the ones commercially available and generally include organic solvents such as propylene glycol, butylenes glycol, ethanol, isopropanol. The active matter in those extracts can vary largely, i.e. in the range of 1 - 30% by weight.

Hair conditioners of the present invention are principally used as leave-in, without rinsing off with water after application, and as well as rinse off. The preferred form of usage is rinse off. Accordingly, further object of the present invention is process for conditioning hair wherein a conditioning composition comprising Polyqauternium-37 and an instable emulsion comprising at least one fatty alcohol and at least one surfactant selected from cationic and non-ionic ones at a weight ratio of cationic and/or non-ionic surfactant to fatty alcohol in the range of 1:2 to 1:10, preferably 1:2 to 1:8 and more preferably 1:2 to 1:5 is applied onto hair after shampooing hair or washing with a cleansing preparation and processed for 1 to 30 min, preferably maximum 20 min and more preferably maximum 10 min and most prerefably maximum 5 min and rinsed off with water.

Further object of the present invention is the process of producing aqueous composition for conditioning hair wherein an instable emulsion comprising at least one fatty alcohol and at least surfactant selected from cationic and non-ionic ones at a weight ratio of cationic and/or non-ionic surfactant to fatty alcohol in the range of 1:2 to 1:10, preferably 1:2 to 1:8 and more preferably 1:2 to 1:5 is first prepared, in the case that during emulsion preparation heat is applied, cooled down to a temperature below 40°C and combined with an aqueous composition comprising poylquaternium 37 at a temperature below 40°C.

The following examples are to illustrate the invention, but not limiting it.

### Example 1

| All values are in % by weight | |
|---|---|
| Ultragel 300 | 1.50 |
| Cetearyl alcohol | 0.60 |
| Cetrimonium chloride | 0.30 |
| Isopropylmyristate | 0.25 |
| Dimethicone | 0.50 |
| Panthenol | 0.50 |
| Glycerin | 2.00 |
| Polyquaternium-11 | 0.30 |
| Lactic acid | q.s to pH 4.0 |
| Fragrance | q.s. |
| Preservative | q.s. |
| Water | to 100 |

The conditioner composition as above was prepared by first combining all ingredients in a vessel other than fragrance, Ultragel 300 (polyquaternium 37) and lactic acid (for pH adjustment) and heating the batch up to 70°C for melting fatty alcohol and homogenizing the mixture for about 5 min at a speed of 2000 rpm and cooling it down to 35°C. Subsequently fragrance and aqueous solution of Ultragel 300 were mixed finally was adjusted with lactic acid. The conditioner thus obtained has a pH value of 4 and has a viscosity of approximately 30,000 mPa.s measured with Brookfield viscosimeter at 20°C with spindle 4 at 10 rpm. The conditioner so obtained is opaque. The above composition was stable at room temperature for a period of longer than 6 months, at 40°C and 50°C for 3 months. The tests were interrupted after the said period at 40 and 50°C.

For comparative purposes two additional compositions were prepared. The composition 1-A was contained 0.6% by weight cetrimonium chloride instead of 0.3% of Example 1 and composition 1-B did not contain any fatty alcohol. The compositions were prepared as described above.

Two half-side comparison tests were carried out. Both compositions (inventive and comparative) were tested against each other in a half-head test with 10 women volunteers having shoulder length fine hair. For this purpose whole head of test person was washed with a commercially available shampoo and the equal amount, approximately 5g, from both conditioners was applied to each side and processed for 5 min. Subsequently rinsed off with water and towel dried and further dried with a hair dryer. In wet and dry stage sensory evaluations were made on hair properties given in Tables I and II.

In the first test, the composition 1-A was tested against composition 1 in order to show the effect of emulsion stability as the emulsion 1-A (all ingredients except Ultragel 300 in combination in water) is a stable emulsion and no separation was observed after storage of 1 month at 40°C. The results are presented in Table I.

**Table I: Sensory evaluation**

| Preferred | | | | |
|---|---|---|---|---|
| | Parameter | Example 1 | Example 1-A | no preference |
| Wet | Easy combing | 4 | 2 | 4 |
| | Smoothness | 4 | 3 | 3 |
| | Roughness | 1 | 3 | 6 |
| | | | | |
| Dry | Easy combing | 4 | 3 | 3 |
| | Smoothness | 3 | 3 | 4 |
| | Elasticity | 8 | 2 | 0 |
| | Volume | 9 | 0 | 1 |
| | Shine | 6 | 2 | 2 |
| | Body | 9 | 0 | 1 |

In the second test the composition 1-B was compared to composition 1 in order to show the effect of instable emulsion. The results are presented in Table II.

**Table II: Sensory evaluation**

| Preferred | | | | |
|---|---|---|---|---|
| | Parameter | Example 1 | Example 1-B | no preference |
| Wet | Easy combing | 9 | 0 | 1 |
| | Smoothness | 8 | 1 | 1 |
| | Roughness | 0 | 3 | 7 |
| | | | | |
| Dry | Easy combing | 9 | 0 | 1 |
| | Smoothness | 7 | 1 | 2 |
| | Elasticity | 9 | 1 | 0 |
| | Volume | 8 | 1 | 1 |
| | Shine | 8 | 1 | 1 |
| | Body | 9 | 0 | 1 |

From the above results, it is clear that the inventive composition according to the present invention gives hair more volume and body and at the same time elasticity, combability and shine of hair is excellent as well.

### Example 2

| All values are in % by weight. | |
|---|---|
| Ultragel 300 | 1.50 |
| Cetearyl alcohol | 0.80 |
| Steartrimonium chloride | 0.20 |
| Isostearlylactylate | 0.25 |
| Glycerin | 2.00 |
| Polyquaternium-10 | 0.30 |
| Cetyl-PG hydroxyethyl palmitamide | 0.20 |
| Avocadin | 0.10 |
| Palmitic acid | 0.02 |
| Citric acid | q.s to pH 4.0 |
| Fragrance | q.s. |
| Preservative | q.s. |
| Water | to 100 |

The test persons used the above composition has evaluated volume, body, elasticity and shine of hair is very much enhanced compared to their daily product and combability to be comparable to that.

Similar results are obtained with the following inventive examples.

### Example 3

| All values are in % by weight. | |
|---|---|
| Basic red 51 | 0.08 |
| Basic brown 16 | 0.10 |
| Basic Violet 2 | 0.008 |
| Ultragel 300 | 1.20 |
| Cetearyl alchol | 1.00 |
| Panthenol | 0.50 |
| Decyl polyglucoside | 0.50 |
| Ceramide of the formula | 0.20 |
| Palmitic acid | 0.02 |
| Avocadin | 0.05 |
| Benzyloxyethanol | 2.00 |
| Glycerin | 2.00 |
| Polyquaternium-10 | 0.20 |
| Citric acid | q.s. to pH 4.6 |
| Fragrance | q.s. |
| Preservative | q.s. |
| Water | to 100 |

The colour obtained with the above conditioner is a red touch on the base colour.

### Example 4

| All values are as % by weight. | |
|---|---|
| Basic red 51 | 0.06 |
| Basic orange 31 | 0.05 |
| Arianor steel blue | 0.02 |
| Ultragel 300 | 1.20 |
| Cetearyl alcohol | 0.70 |
| Behentrimonium chloride | 0.30 |
| Panthenol | 0.70 |
| Aloe vera extract | 1.00 |
| Polyethylene glycol 200 | 1.00 |
| Polyquaternium-10 | 0.20 |
| Citric acid | q.s. to pH 3.5 |
| Fragrance | q.s. |
| Preservative | q.s. |
| Water | to 100 |

Intensive red touch is obtained on a dark blond hair.

### Example 5

| All values are as % by weight. | |
|---|---|
| Ultragel 300 | 1.50 |
| Cetearyl alcohol | 1.00 |
| Panthenol | 0.70 |
| Ceteareth-20 | 0.50 |
| Isopropyl palmitate | 0.30 |
| Sandelwood extract | 1.00 |
| Polyethylene glycol 200 | 1.00 |
| Octylmethoxycinnamate | 0.20 |
| Citric acid | q.s. to pH 4.5 |
| Fragrance | q.s. |
| Preservative | q.s. |
| Water | to 100 |

### Example 6

| All values are as % by weight. | |
|---|---|
| Arianor madder red | 0.0001 |
| Basic violet 2 | 0.000045 |
| Arianor steel blue | 0.002 |
| Ultragel 300 | 1.20 |
| Ctearyl alcohol | 0.80 |
| Behentrimoniumchloride | 0.30 |
| Dioleoylethyldimethyl ammonium methosulfate | 0.50 |
| Panthenol | 0.70 |
| PEG-60 hydrogenated castor oil | 0.10 |
| Aloe vera extract | 1.00 |
| Polyethylene glycol 200 | 1.00 |
| Citric acid | q.s. to pH 4.2 |
| Fragrance | q.s. |
| Preservative | q.s. |
| Water | to 100 |

Platin blond appearance is observed upon application on a gray hair. The composition is as well applied to bleached hair and anti yellow effect is observed. The composition is especially suitable for dry hair as a moisturising conditioner as well in addition to its excellent volume and body giving properties.

## Claims

1. Conditioning composition for hair **characterised in that** it comprises Polyquaternium-37 at a concentration of 0.1 to 5% by weight, calculated to total composition, and an instable emulsion comprising at least one fatty alcohol at a concentration of 0.1 to 2.5% by weight, calculated to total composition, and at least one surfactant selected from non-ionic and cationic ones at a weight ratio of cationic and/or non-ionic surfactants to fatty alcohol in the range of 1: 2 to 1:10, wherein cationic surfactants are selected from compounds according to general formula 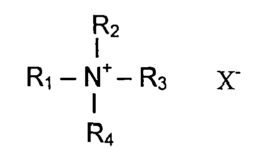 where R₁ is a saturated or unsaturated, branched or non-branched alkyl chain with 8-22 C atoms or
R₅ CO NH (CH₂)ₙ
where R₅ is saturated or unsaturated, branched or non-branched alkyl chain with 7-21 C atoms and n has value of 1 - 4,
or
R₆ CO O (CH₂)ₙ
where R₆ is saturated or unsaturated, branched or non-branched alkyl chain with 7-21 C atoms and n has value of 1 - 4, and
R₂, R₃ and R₄ are H or lower alkyl or hydroxyalkyl or polyhydroxyalkyl with 1 to 4 C atoms and X is chloride, bromide, methosulfate.

2. Composition according to claim 1 nonionic surfactants are selected from C₁₀-C₂₂ fatty alcohol ethoxylates, alkylpolyglucosides and sorbitan esters.

3. Composition according to claims1 and 2 **characterised in that** it comprises at least one conditioning agent selected from quaternary ammonium compounds of the general formula 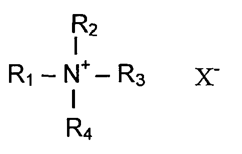 where R₁ is a saturated or unsaturated, branched or non-branched alkyl chain with 8-22 C atoms or
R₅ CO NH (CH₂)ₙ
where R₅ is saturated or unsaturated, branched or non-branched alkyl chain with 7-21 C atoms and n has value of 1 - 4,
or
R₆ CO O (CH₂)ₙ
where R₆ is saturated or unsaturated, branched or non-branched alkyl chain with 7-21 C atoms and n has value of 1 - 4, and
R₂ is unsaturated or saturated, branched or linear alkyl chain with 8 to 22 C atoms or
R₅ CO NH (CH₂)ₙ
or
R₆ CO O (CH₂)ₙ
where R₅, R₆ and n are same as above, and
R₃ and R₄ are H or lower alkyl or hydroxyalkyl or polyhydroxyalkyl with 1 to 4 C atoms and X is chloride, bromide, methosulfate,
cationic polymers, silicone compounds, natural or synthetic oils, non-ionic conditioning agents and hair restructuring compounds.

4. Composition according to any of the preceding claims **characterised in that** it comprises at least one ester of aliphatic linear or branched carboxylic acid with a primary or secondary linear or branched alcohol at a concentration of 0.01 to 5% by weight, calculated to total composition.

5. Composition according to any of the preceding claims **characterised in that** it comprises ate least one direct acting dyestuff.

6. Composition according to any of the preceding claims **characterised in that** it comprises ate least one organic solvent.

7. Composition according to any of the preceding claims **characterised in that** it comprises ate least one UV filter.

8. Composition according to any of the preceding claims **characterised in that** it has a pH between 2 and 7.

9. Use of a composition according to any of the claims 1 to 8 for enhancing body and volume of human hair.

10. Process for conditioning hair wherein a composition according to claims 1 to 8 is applied onto hair after shampooing or washing with a cleansing preparation and processed for 1 to 30 min and rinsed off with water.

11. Process for producing composition according to claims 1 to 8 **characterised in that** first an instable emulsion is prepared by combining at least one fatty alcohol and at least one surfactant selected from cationic and non-ionic ones at a weight ratio of cationic and/or non-ionic surfactants to fatty alcohol in the range of 1: 2 to 1:10 and cooled down to a temperature below 40°C in the case heat is applied to for emulsification and mixed with an aqueous composition comprising polyquaternium 37 at a temperature below 40°C.
